Europäisches Patentamt

European Patent Office

Office européen des brevets

(19)

(11) Numéro de publication : **0 055 659**
**B1**

(12) FASCICULE DE BREVET EUROPÉEN

(45) Date de publication du fascicule du brevet :
05.06.85

(51) Int. Cl.⁴ : **B 01 J 23/26,** B 01 J 35/10,
B 01 J 21/18, C 07 C 19/08,
C 07 C 17/20

(21) Numéro de dépôt : 81402026.9

(22) Date de dépôt : 18.12.81

(54) Catalyseurs de fluoruration en phase gazeuse des hydrocarbures chlorés et chlorofluorés aliphatiques, à base d'oxyde de chrome déposé sur charbon actif, et procédés de fluoruration utilisant ces catalyseurs.

(30) Priorité : 29.12.80 FR 8027661

(43) Date de publication de la demande :
07.07.82 Bulletin 82/27

(45) Mention de la délivrance du brevet :
05.06.85 Bulletin 85/23

(84) Etats contractants désignés :
BE DE FR GB IT NL

(56) Documents cités :
FR-A- 1 309 786
FR-A- 2 111 865
GB-A- 943 627
US-A- 2 982 000
US-A- 3 600 450

(73) Titulaire : ATOCHEM
12/16, allée des Vosges
F-92400 Courbevoie (FR)

(72) Inventeur : Foulletier, Louis
5, chemin Croix-Berthet
F-69600 Oullins (FR)

(74) Mandataire : Rochet, Michel et al
ATOCHEM Département Propriété Industrielle Cedex
22
F-92091 Paris la Défense (FR)

**0 055 659**

## Description

La présente invention concerne des catalyseurs améliorés pour la fluoruration en phase gazeuse par l'acide fluorhydrique des hydrocarbures chlorés ou chlorofluorés aliphatiques, constitués par des oxydes de chrome déposés sur charbon actif. Elle concerne également des procédés de fluoruration de ces dérivés chlorés ou chlorofluorés utilisant ces catalyseurs dans des réacteurs à lit fluidisé.

De très nombreux catalyseurs ont été proposés pour ces réactions de substitution d'atomes de chlore par des atomes de fluor, en phase gazeuse. Ce sont le plus souvent des oxydes ou des halogénures de chrome, d'aluminium, de cobalt, de fer, de titane, de nickel, de cuivre, de palladium ou de zirconium, utilisés tels quels ou sur des supports divers, comme les charbons actifs et les alumines.

C'est ainsi que le brevet des Etats-Unis no 2 458 551 décrit des catalyseurs à base de trifluorure de chrome, déposé sur charbon actif ou sur fluorure de cobalt.

Dans le brevet des Etats-Unis no 3 258 500 est revendiqué un catalyseur à base d'oxyde de chrome trivalent, anhydre, non supporté, préparé par réduction du trioxyde de chrome à l'éthanol et activé par chauffage à 400-600 °C.

D'autres brevets, comme les demandes japonaises no 70 116 696 et no 74 131 610, enseignent la réduction du trioxyde de chrome par des aldéhydes ou de l'hydrazine en vue de la préparation de catalyseurs de fluoruration.

Le brevet britannique no 896 068 et le brevet des Etats-Unis no 35157 707 décrivent la réduction à l'hydrogène de CrO₃ déposé sur alumine activée, pour préparer des catalyseurs de fluoruration du trichlorotrifluoréthane, du dichlorotétrafluoréthane, du dichlorotétrafluoréthane ou de l'hexachloréthane.

Le brevet des Etats-Unis no 2 892 000 décrit la préparation du fluorure de vinyle et du difluoro-1,1-éthane par addition d'acide fluorhydrique sur l'acétylène, en présence de catalyseurs obtenus par imprégnation de charbon de bois au moyen d'une solution de trioxyde de chrome et réduction à l'hydrogène à 400 °C. Dans un exemple de ce brevet, l'étape de réduction à l'hydrogène est apparemment supprimée.

Tous ces catalyseurs de l'art antérieur conviennent plus ou moins bien à la fluoruration en phase gazeuse des chloroalcanes ou des chlorofluoroalcanes dans des réacteurs à lit fixe. Ils sont par contre très mal adaptés à la fluoruration dans des réacteurs à lit fluidisé, qui exigent des particules de forme régulière et de granulométrie homogène. Le simple broyage du catalyseur, suivi d'un tamisage pour sélectionner les particules de taille convenable, donne des grains de forme irrégulière et conduit à des déchets importants de catalyseur.

La demanderesse a découvert que les catalyseurs de fluoruration en phase gazeuse s'empoisonnent principalement par la formation de goudrons à leur surface. L'emploi de réacteurs à lit fluidisé, provoquant l'abrasion des grains de catalyseur, permet d'éliminer ces goudrons et de maintenir l'activité du catalyseur. Celui-ci se consomme uniquement par attrition, et il n'est plus besoin d'arrêter l'installation pour recharger le réacteur en catalyseur.

Les catalyseurs de l'art antérieur présentent également au moins l'un des inconvénients suivants :

— faible taux de transformation de l'acide fluorhydrique
— faible productivité
— faible sélectivité
— taux élevé d'isomères asymétriques dans la production du trichlorotrifluoréthane et du dichlorotétrafluoréthane.

Selon l'invention on remédie à tous ces inconvénients en préparant des catalyseurs de fluoruration en phase gazeuse par imprégnation, au moyen d'une solution aqueuse de trioxyde de chrome CrO₃, d'un charbon actif possédant une surface spécifique totale supérieure à 1 000 m²/g et possédant une mésoporosité et une macroporosité élevées, et en séchant le catalyseur à environ 150 °C.

La mésoporosité, définie par la surface des pores de 40 à 50 Å de rayon, doit être supérieure à 5 m²/g. La macroporosité, définie par la surface des pores dont le rayon est égal ou supérieur à 250 Å, doit être supérieure à 2 m²/g. Ces valeurs sont particulièrement critiques pour obtenir des taux de transformation élevés de l'acide fluorhydrique.

La majeure partie du trioxyde de chrome CrO₃ utilisé à l'imprégnation est réduite, dans le catalyseur terminé, à l'état de sesquioxyde Cr₂O₃ par le charbon actif lui-même, sans qu'il soit besoin de faire appel à un autre réducteur. La réaction est très exothermique, mais elle peut être contrôlée facilement, même à grande échelle. Cette réduction du trioxyde de chrome par le charbon actif a de plus l'avantage d'augmenter la macroporosité du charbon actif.

Les catalyseurs selon l'invention contiennent de préférence de 0,5 à 2,8 atomes-gramme de chrome par litre et se présentent sous forme de particules de 100 à 3 000 µm de diamètre. Ils peuvent être utilisés tels quels après séchage ou soumis à une activation par l'acide fluorhydrique en fin de séchage, ce qui provoque la formation d'un peu de trifluorure de chrome à la surface des grains.

Ces catalyseurs conviennent particulièrement à la fluoruration en phase gazeuse des dérivés chlorés

2

0 055 659

ou chlorofluorés du méthane et de l'éthane. Ils sont moins bien adaptés à la fluoruration des dérivés halogénés, des cétones, comme l'hexachloracétone, ou des nitriles, comme le trichloroacétonitrile.

Les exemples suivants, donnés à titre non limitatif, illustrent divers modes de préparation des catalyseurs selon l'invention et l'utilisation de ces catalyseurs dans diverses réactions de fluoruration.

## Exemple 1

On prépare une solution de 1,0 mole de trioxyde de chrome dans 300 cm$^3$ d'eau et on en imprègne 1 litre de charbon actif végétal présentant les caractéristiques suivantes :

| | |
|---|---|
| Densité | 0,586 |
| Granulométrie | 1,8 mm |
| Surface spécifique totale | 1 096 m$^2$/g |
| Surface des pores $\geqslant$ 250 Å | 2,16 m$^2$/g |
| Surface des pores 250-50 Å | 16,42 m$^2$/g |
| Surface des pores 50-40 Å | 8,01 m$^2$/g. |

Le charbon actif adsorbe la totalité de la solution. Le catalyseur est séché en lit fluidisé par de l'air à 150 °C. L'analyse du catalyseur sec montre que la plus grande partie du chrome est à l'état trivalent.

Ce catalyseur est utilisé pour la fluoruration en lit fluidisé du trichlorotrifluoréthane, dans les conditions suivantes :

| | |
|---|---|
| Rapport molaire HF/C$_2$Cl$_3$F$_3$ | 1,11/1 |
| Débit | 14,4 moles/h/l |
| Température | 412 °C. |

Le taux de transformation de l'acide fluorhydrique est de 71 %. Les taux de transformation du trichlorotrifluoréthane sont de :

68 % en dichlorotétrafluoréthane, contenant 89 % d'isomère symétrique
  5 % en monochloropentafluoréthane.

## Exemple 2

Avec le charbon actif de l'exemple 1, on prépare dans les mêmes conditions un catalyseur contenant 2,0 atomes/g de chrome par litre.

Ce catalyseur est utilisé pour la fluoruration en lit fluidisé du trichlorotrifluoréthane dans les conditions de l'exemple 1.

Le taux de transformation de l'acide fluorhydrique est de 83 %. Les taux de transformation du trichlorotrifluoréthane sont de :

72 % en dichlorotétrafluoréthane, contenant 82 % d'isomère symétrique
10 % en monochloropentafluoréthane.

## Exemple 3 (Exemple comparatif)

Comme dans l'exemple 1, on prépare un catalyseur contenant 1 atome/g de chrome par litre, mais en utilisant comme support un charbon actif qui ne présente pas les caractéristiques voulues de mésoporosité et de macroporosité, et par conséquent ne convient pas à la préparation des catalyseurs selon l'invention.

Ce charbon actif présente les caractéristiques suivantes :

| | |
|---|---|
| Densité | 0,659 |
| Surface spécifique totale | 1 347 m$^2$/g |
| Surface des pores $\geqslant$ 250 Å | 1,41 m$^2$/g |
| Surface des pores 250-50 Å | 8,7 m$^2$/g |
| Surface des pores 50-40 Å | 4,7 m$^2$/g. |

Ce catalyseur est utilisé, dans les conditions de l'exemple 1, pour la fluoruration en lit fluidisé du trichlorotrifluoréthane.

Le taux de transformation de l'acide fluorhydrique n'est que de 51 %. Les taux de transformation du trichlorotrifluoréthane sont de :

63 % en dichlorotétrafluoréthane, contenant 78 % d'isomère symétrique
  2 % en monochloropentafluoréthane.

3

## Exemple 4

On utilise le catalyseur de l'exemple 2 pour fluorer du monochlorotrifluorométhane dans les conditions suivantes :

| | |
|---|---|
| Rapport molaire HF/CF$_3$Cl | 1,55/1 |
| Temps de contact | 3,72 secondes |
| Température | 450 °C. |

Le taux de transformation de l'acide fluorhydrique est de 47 %. Le taux de transformation du chlorotrifluorométhane en tétrafluorométhane est de 73 %

Par comparaison, un catalyseur préparé par imprégnation de charbon actif avec du trifluorure de chrome, selon les indications du brevet des Etats-Unis no 2 458 551, donne, avec des conditions de marche beaucoup plus sévères :

| | |
|---|---|
| Rapport molaire HF/CF$_3$Cl | 3,1/1 |
| Temps de contact | 12 secondes |
| Température | 880 °C. |

un taux de transformation de l'acide fluorhydrique de seulement 28 % et un taux de transformation du CF$_3$Cl en Cf$_4$ de 88,5 %.

Un tel catalyseur a de plus une durée de vie extrêmement brève, car il supporte mal des températures supérieures à 500 °C.

## Exemple 5

On prépare un catalyseur analogue à celui de l'exemple 1, mais contenant 2,5 atomes/g de chrome par litre, et on l'active par un traitement à l'acide fluorhydrique pendant 3 heures à 150 °C en fin de séchage.

Ce catalyseur est utilisé pour la fluoruration du dichlorodifluorométhane dans une installation continue, comprenant deux réacteurs à lit fluidisé montés en série.

Le premier réacteur, maintenu à la température de 430 °C, est alimenté par un mélange de 17,9 moles d'acide fluorhydrique, 16,1 moles de chlorotrifluorométhane et 1,8 mole de dichlorodifluorométhane recyclés. Le temps de contact est de 3 secondes.

Les effluents de ce premier réacteur sont mélangés avec 11,0 moles de dichlorodifluorométhane et envoyés dans le second réacteur, porté à la température de 450 °C. Le temps de contact dans ce réacteur est de 4 secondes.

Les effluents contiennent :

| | |
|---|---|
| Trichlorofluorométhane (formé par dismutation) | 1,2 % en moles |
| Dichlorodifluorométhane non transformé | 6,2 % en moles |
| Chlorotrifluorométhane | 71,8 % en moles |
| Tétrafluorométhane | 20,8 % en moles. |

Le taux global de transformation de l'acide fluorhydrique est de 94,9 %. On extrait de l'installation la totalité du tétrafluorométhane et une partie du chlorotrifluorométhane, le reste étant recyclé à l'entrée du réacteur 1. Le dichlorodifluorométhane non transformé est recyclé à l'entrée du réacteur 2.

## Revendications

1. Catalyseurs pour la fluoruration en phase gazeuse des hydrocarbures chlorés et chlorofluorés aliphatiques, à base d'oxyde de chrome déposé sur charbon actif par imprégnation au moyen d'une solution aqueuse de trioxyde de chrome, caractérisés en ce qu'ils sont obtenus à partir d'un charbon actif présentant une surface spécifique totale supérieure à 1 000 m$^2$/g, une surface des pores de 40 à 50 Å de rayon supérieure à 5 m$^2$/g et une surface des pores qui ont un rayon égal ou supérieur à 250 Å supérieure à 2 m$^2$/g, l'imprégnation étant suivie d'un séchage à une température d'environ 150 °C.

2. Catalyseurs selon la revendication 1, caractérisés en ce qu'ils contiennent de 0,5 à 2,8 atomes/g de chrome par litre.

3. Catalyseurs selon l'une des revendications 1 ou 2, caractérisés en ce qu'ils sont sous forme de particules de 100 μm à 3 000 μm de diamètre.

4. Catalyseurs selon l'une des revendications 1 à 3, caractérisés en ce qu'ils sont activés avant emploi par un traitement à l'acide fluorhydrique anhydre.

5. Procédé de fluoruration en phase gazeuse des hydrocarbures chlorés et chlorofluorés aliphatiques, caractérisé par l'utilisation dans un réacteur à lit fluidisé d'un catalyseur selon l'une ou l'autre des revendications 1 à 4.

6. Procédé de fluoruration en phase gazeuse du dichlorodifluorométhane, caractérisé par l'utilisation de deux réacteurs à lit fluidisé, montés en série et garnis d'un catalyseur selon l'une des revendications 1 à 4, le premier réacteur étant alimenté en acide fluorhydrique et en chlorotrifluorométhane et dichlorodifluorométhane recyclés, et le second réacteur étant alimenté par les effluents du premier additionnés de dichlorodifluorométhane frais.

## Claims

1. Catalysts for the gas phase fluoridation of chlorinated and chlorofluorinated aliphatic hydrocarbons, based on chromium oxide deposited on activated charcoal by impregnation with an aqueous solution of chromium trioxide, characterised in that they are obtained from an activated charcoal having a total specific surface area greater than 1 000 $m^2/g$, a surface area of pores having a radius of from 40 to 50 Å which is greater then 5 $m^2/g$ and a surface area of pores having a radius equal to or greater than 250 Å which is greater than 2 $m^2/g$, the impregnation being followed by drying at a temperature of approximately 150 °C.

2. Catalysts according to Claim 1, characterised in that they contain from 0.5 to 2.8 gram-atoms of chromium per litre.

3. Catalysts according to either of Claims 1 or 2, characterised in that they are in the form of particles 100 μm to 3,000 μm in diameter.

4. Catalysts according to any one of Claims 1 to 3, characterised in that they are activated before use by a treatment with anhydrous hydrofluoric acid.

5. Process for the gas phase fluoridation of chlorinated and chlorofluorinated aliphatic hydrocarbons, characterised by the use in a fluidized bed reactor of a catalyst according to any one of Claims 1 to 4.

6. Process for the gas phase fluoridation of dichlorodifluoromethane characterised by the use of two fluidized bed reactors mounted in series and provided with a catalyst according to one of Claims 1 to 4, the first reactor being fed with hydrofluoric acid and with chlorotrifluoromethane and dichlorodifluoromethane which are recycled, and the second reactor being fed with the effluents from the first to which fresh dichlorodifluoromethane has been added.

## Patentansprüche

1. Katalysatoren zur Fluorierung von aliphatischen Chlor- und Chlorfluorkohlenwasserstoffen in der Gasphase auf Basis von Chromoxid, das durch Imprägnierung mit einer wässrigen Lösung von Chromtrioxid auf Aktivkohle abgelagert wird, dadurch gekennzeichnet, daß sie, ausgehend von einer Aktivkohle, mit einer spezifischen Gesamtoberfläche der Poren mit einem Radius zwischen 40 und 50 Å von über 5 $m^2/g$ und einer Oberfläche der Poren mit einem Radius von 250 Å oder darüber von mehr als 2 $m^2/g$ erhalten werden, wobei anschließend an die Imprägnierung eine Trocknung bei einer Temperatur von etwa 150 °C erfolgt.

2. Katalysatoren nach Anspruch 1, dadurch gekennzeichnet, daß sie zwischen 0,5 und 2,8 gramm-Atom Chrom pro Liter enthalten.

3. Katalysatoren nach Anspruch 1 oder 2, dadurch gekennzeichnet, daß sie in Form von Teilchen mit einem Durchmesser zwischen 100 μm und 3 000 μm vorliegen.

4. Katalysatoren nach einem der Ansprüche 1 bis 3, dadurch gekennzeichnet, daß sie vor dem Gebrauch durch eine Behandlung mit wasserfreier Fluorwasserstoffsäure aktiviert werden.

5. Verfahren zur Fluorierung von aliphatischen Chlor- und Chlorfluorkohlenwasserstoffen in der Gasphase, dadurch gekennzeichnet, daß in einem Fließbettreaktor ein Katalysator nach einem der Ansprüche 1 bis 4 eingesetzt wird.

6. Verfahren zur Fluorierung von Dichlordifluormethan in der Gasphase, gekennzeichnet durch die Verwendung von zwei hintereinander geschalteten Fießbettreaktoren, die mit einem Katalysator nach einem der Ansprüche 1 bis 4 beschichkt sind, wobei in den ersten Reaktor Fluorwasserstoff, Chlortrifluormethan und Dichlordifluormethan aus der Rückführung und in den zweiten Reaktor die Ströme aus dem ersten unter Zugabe von frischem Dichlordifluormethan eingesetzt werden.